# EUROPEAN PATENT APPLICATION

(11) **EP 3 838 040 A1**
(43) Date of publication of application: **23.06.2021**
(21) Application number: 20215887.9
(22) Date of filing: 21.12.2020
(51) Int. Cl.: A41G 5/00, C09J 105/00

(54) **GLUE-FREE ADHESIVE EYELASHES**

(30) Priority: 20.12.2019 US 201962951238 P; 03.12.2020 US 202017110982
(71) Applicant: America International Industries, Los Angeles CA 90040 (US)
(72) Inventor: Yu, Hao, Qingdau, 266071 (CN)
(74) Representative: Engstle, Verena

(57) **Abstract**

An aqueous base, glue-free composition is comprised of an effective amount of ingredients (e.g., arabic gum, casein and polyvinylpyrrolidone) which exhibit sticky adhesion properties which do not tightly bind so as to allow an eyelash extension to be removably attached to a natural eyelash without the need for use of adhesive remover composition.

## Description

### Cross Reference to Related Applications

This application is a non-provisional application which claims priority from U.S. Ser. No. 62/951,238, filed 12/20/2019, the disclosure of which is specifically incorporated by reference herein in its entirety.

### Field of the Invention

The present invention is in the field of eyelash enhancements.

### Background of the Invention

It is known that eyelash extensions can use magnets to attach upper and under eyelashes to a natural eyelash. It is also known that magnetic eyeliner can be used to attach an eyelash extension to a natural eyelash. Finally, it is also known that adhesive components (glue) can be used to attach an eyelash extension to a natural eyelash.

The present invention seeks to improve upon the existing art by offering a new and better alternative way to attach eyelash extensions to natural eyelashes.

### SUMMARY OF THE INVENTION

The present invention is generally directed to a glue-free adhesive for eyelashes and a method for securing eyelashes with a glue-free adhesive. More particularly, the present invention uses effective amounts of arabic gum, casein and polyvinylprrolidone in water to formulate a glue-free adhesive which allows eyelash attachments to be removably attached to eyelashes without the need for use of an adhesive remover to remove the eyelash attachments.

Accordingly, it is an object of the present invention to provide an improved eye-lash enhancement which uses a glue-free adhesive.

This and further objects and advantages will be apparent to those skilled in the art in connection with the drawings and the detailed description of the invention set forth below.

### DETAILED DESCRIPTION OF THE INVENTION

In accordance with the present invention, a glue-free composition is used to attach one or more eyelash strip lashes to a natural eyelash. In the context of the present invention, "glue-free" means that the composition exhibits sticky adhesion properties which allow an eyelash strip lash to be removably attachable to a natural eyelash such that such extension can be readily removed without the need for using an adhesive remover composition to effectuate such removal.

The glue-free composition of the present invention can be used to attach an upper strip lash, or an under strip lash, or both, to a natural eyelash. To effectuate such attachment, the glue-free composition can be applied to the attachment(s) or to the natural eyelash, or to both, and then the attachment(s) is pressed against the natural eyelash. Because the glue-free composition exhibits sticky adhesive properties, it allows the attachment to remain attached (or stuck) once the attachment has been applied; however, because the glue-free composition does not cause the attachment to bind together tightly with the natural eyelash, the attachment can be removed, without the need for use of an adhesive or glue remover composition, by simply pulling the attachment away from the eye lash in much the same manner as a Post-It® note can be applied to a surface and yet readily removed from the surface.

According to the present invention, a composition, in particular a glue-free composition comprises:
an effective amount of arabic gum;
an effective amount of casein;
an effective amount of polyvinylpyrrolidone; and
water;
wherein the composition will allow an adhesive to be removably attachable to an eyelash.

According to a preferred embodiment of the composition, the effective amount of arabic gum is approximately 25 weight percent.

According to another preferred embodiment of the composition, the effective amount of casein is approximately 7.5 weight percent.

According to another preferred embodiment of the composition, wherein the effective amount of polyvinylpyrrolidone is approximately 7.5 weight percent.

According to another preferred embodiment of the composition, the effective amount of water is approximately 60 weight percent.

According to another preferred embodiment of the composition, the effective amount of arabic gum is between approximately 23 to approximately 28 weight percent, the effective amount of casein is between approximately 5 to approximately 8.5 weight percent, the effective amount of polyvinylpyrrolidone is between approximately 6 to approximately 8 weight percent and the effective amount of water is between approximately 58 to approximately 67 weight percent.

According to another preferred embodiment of the composition,
the effective amount of arabic gum is comprised of between 23-28 weight percent of arabic gum;
the effective amount of casein is comprised of between 5-8.5 weight percent of casein;
the effective amount of polyvinylpyrrolidone is comprised of between 6-8 weight percent of polyvinylpyrrolidone; and
water comprised between 58-67 weight percent of the composition.

Furthermore, according to the present invention, a composition, in particular a composition including any of the features described before, comprises an effective amount of arabic gum or its equivalent;
an effective amount of casein or its equivalent;
an effective amount of polyvinylpyrrolidone or its equivalent; and
water;
wherein the composition exhibits sticky adhesion properties which allow an eyelash extension to be removably attachable to a natural eyelash such that said extension can be readily removed without the need for using an adhesive remover composition to effectuate such removal.

A method according to the present invention for removably applying an eyelash extension to a natural eyelash comprises applying an effective amount of a glue-free composition to at least one of the eyelash extension and the natural eyelash and then pressing the eyelash extension and the natural eyelash together and then removing the eyelash extension from the natural eyelash without the need for an adhesive remover by pulling the two apart, wherein the glue-free composition is comprised of an effective amount of ingredients in an aqueous base which exhibits sticky adhesion properties which do not tightly bind the eyelash extension to the natural eyelash.

Without intending to limit the present invention to the exact composition of the following formula, it has been found that an especially preferred composition according to the present invention is comprised of between approximately 23-28 weight percent arabic gum (CAS No. 9000-01-5), between approximately 5-8.5 weight percent casein (CAS No. 9000-71-9), between approximately 6-8 weight percent polyvinylpyrrolidone (CAS No. 9003-39-8) and between approximately 58-67 weight percent water (CAS No. 7732-18-5), with one preferred embodiment having a formulation of 25.0 weight percent arabic gum, 7.5 weight percent casein, 7.5 weight percent polyvinylpyrrolidone and 60 weight percent water. It is believed that the weight percentages of the foregoing ingredients may be varied, while additional inert ingredients may be added, and equivalents to such ingredients may be substituted (in appropriate weight percentages), to get substantially the same result, as would be apparent to one skilled in the art exercising routine experimentation after studying the disclosure of the present invention.

Accordingly, it will be readily apparent to those skilled in the art that still further changes and modifications in the actual concepts described herein can readily be made without departing from the spirit and scope of the disclosed inventions.

## Claims

1. (original): A composition, comprising:
an effective amount of arabic gum;
an effective amount of casein;
an effective amount of polyvinylpyrrolidone; and
water;
wherein the composition will allow an adhesive to be removably attachable to an eyelash.

2. (original): The composition of claim 1, wherein the effective amount of arabic gum is approximately 25 weight percent.

3. (original): The composition of claim 1 or 2, wherein the effective amount of casein is approximately 7.5 weight percent.

4. (original): The composition of any of the preceding claims, wherein the effective amount of polyvinylpyrrolidone is approximately 7.5 weight percent.

5. (original): The composition of any of the preceding claims, wherein the effective amount of water is approximately 60 weight percent.

6. (original): The composition of any of the preceding claims, wherein the effective amount of arabic gum is between approximately 23 to approximately 28 weight percent, the effective amount of casein is between approximately 5 to approximately 8.5 weight percent, the effective amount of polyvinylpyrrolidone is between approximately 6 to approximately 8 weight percent and the effective amount of water is between approximately 58 to approximately 67 weight percent.

7. (original): The composition of any of the preceding claims, wherein:
the effective amount of arabic gum is comprised of between 23-28 weight percent of arabic gum;
the effective amount of casein is comprised of between 5-8.5 weight percent of casein;
the effective amount of polyvinylpyrrolidone is comprised of between 6-8 weight percent of polyvinylpyrrolidone; and
water comprised between 58-67 weight percent of the composition.

8. (original): A composition, in particular according to any of the preceding claims, comprising:
an effective amount of arabic gum or its equivalent;
an effective amount of casein or its equivalent;
an effective amount of polyvinylpyrrolidone or its equivalent; and
water;
wherein the composition exhibits sticky adhesion properties which allow an eyelash extension to be removably attachable to a natural eyelash such that said extension can be readily removed without the need for using an adhesive remover composition to effectuate such removal.

9. (original): A method for removably applying an eyelash extension to a natural eyelash comprising applying an effective amount of a glue-free composition to at least one of the eyelash extension and the natural eyelash and then pressing the eyelash extension and the natural eyelash together and then removing the eyelash extension from the natural eyelash without the need for an adhesive remover by pulling the two apart, wherein the glue-free composition is comprised of an effective amount of ingredients in an aqueous base which exhibits sticky adhesion properties which do not tightly bind the eyelash extension to the natural eyelash.
